(19) Europäisches Patentamt
European Patent Office
Office européen des brevets

(11) **EP 3 936 046 A1**

(12) **EUROPEAN PATENT APPLICATION**

(43) Date of publication:
**12.01.2022 Bulletin 2022/02**

(51) Int Cl.:
***A61B 5/11*** (2006.01)    ***A61B 5/12*** (2006.01)
***G16H 50/20*** (2018.01)

(21) Application number: **20185050.0**

(22) Date of filing: **09.07.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **HIWALE, Sujitkumar Sureshrao**
**5656 AE Eindhoven (NL)**

• **CHAKRABARTI, Biswaroop**
**5656 AE Eindhoven (NL)**
• **RADHAKRISHNAN, Ravindranath**
**5656 AE Eindhoven (NL)**
• **JOHNSON, Mark Thomas**
**5656 AE Eindhoven (NL)**
• **BERA, Deep**
**5656 AE Eindhoven (NL)**

(74) Representative: **Philips Intellectual Property & Standards**
**High Tech Campus 52**
**5656 AG Eindhoven (NL)**

(54) **SYSTEMS AND METHODS FOR ASSESSING EAR PATHOLOGIES IN A SUBJECT**

(57)    The invention provides a system for assessing ear pathologies in a subject. The system includes an earplug configured to be at least partially inserted in the ear canal of an ear of a subject. The earplug comprises a pressure sensor adapted to generate passive pressure signals representative of passive pressure changes within the ear canal induced by maneuvers performed by the subject, wherein characteristics of the passive pressure signals are representative of one or more ear pathologies.

FIG. 2

EP 3 936 046 A1

**Description**

FIELD OF THE INVENTION

The invention relates to the field of clinical ear examination, and more specifically to investigation of ear pathologies.

BACKGROUND OF THE INVENTION

The investigation and monitoring of ear health is field undergoing continuous development. Pathologies of the external ear, the tympanic membrane and the middle ear are often important cause for a visit to a doctor, especially in the pediatric age group. Pathologies of ear include wax collection in the external ear to significant deafness. For a primary care physician, it is important to properly assess these pathologies, and either treat or refer them to respective specialists.

Currently, ear investigations often involve the use of an otoscope and pneumatic otoscopy for performing the ear examination. These methods are widely used, but are time consuming and the quality of the results is heavily dependent upon the skill of the operator. As such, no simple method exists for a quick detection of common ear pathologies.

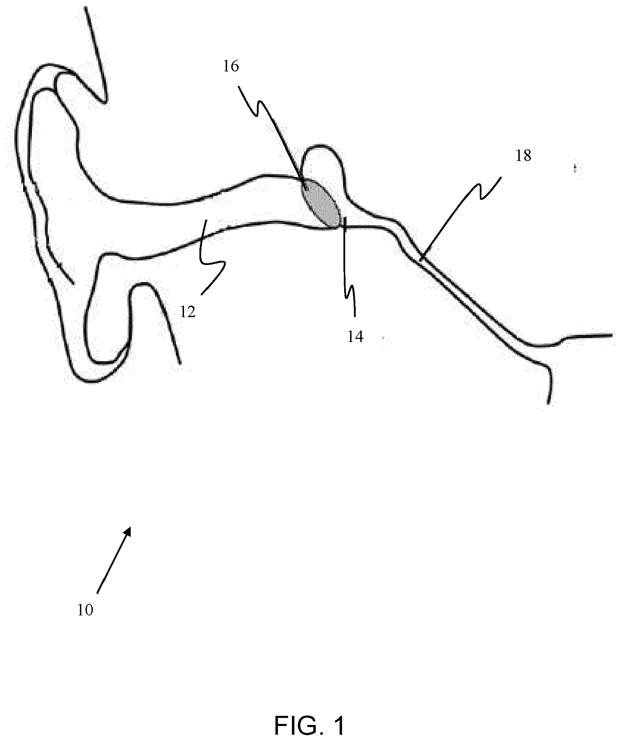

Figure 1 shows a schematic representation of an ear 10 of a subject, which is divided into three parts: the external ear 12, the middle ear 14 and the inner ear. Common pathologies of the external ear include infections and wax collection. The external ear includes the pinna, the ear canal and the outer layer of the eardrum. The external ear canal is approximately 2.5 cm in length with diameter of 0.7 cm.

The middle ear is an air-filled cavity between the eardrum 16 and inner ear. It includes the tympanic membrane and three small bones (ossicles). Unlike the external ear canal, however, the air of the middle ear is not in direct contact with the atmosphere. The middle ear cavity is connected to the back of the nasopharynx via an auditory tube names the Eustachian tube 18.

Common pathologies of the middle ear include perforation of tympanic membrane (ear drum) 18, infection in the middle ear and pathologies of ossicular system, which may lead to hearing loss.

The inner ear is a hallow cavity in the temporal bone and consists of the cochlea, which is an organ dedicated to hearing, and the vestibular system, which is an organ dedicated to balance. Pathologies of the inner ear typically result in hearing loss.

The pressure in the middle ear is maintained very close to atmospheric pressure by various physiological mechanisms including airflow through the Eustachian tube 18 as discussed in H. W. Pau et al. "Pressure changes in the human middle ear without opening the Eustachian tube," Acta Oto-Laryngologica, vol. 129, no. 11, pp. 1182-1186, Jan. 2009. The Eustachian tube comprises outer bony parts (close to the middle ear cavity) and inner cartilaginous parts (close to nasopharynx) with the walls of the cartilaginous part formed partially by cartilage and partially by a fibrous membrane allowing the tube to be collapsed. Normally, the Eustachian tube is collapsed, but it gapes open when the subject is swallowing or performing a maneuvers that creates a positive pressure in the nasal cavity. The Eustachian tube is known to be involved in various cochleavetibular diseases.

As discussed above, a physical examination of the ear usually involves inspection of the ear cavity using a specialized tool known as otoscope, which is time consuming and difficult to use situations where the subject has a painful conditions. Secondly, it is not possible to detect all the pathologies in a single examination. A further method to study the tympanic membrane properties is using pneumatic otoscopy, which involves observation of the degree of eardrum mobility in response to manually introduced positive and negative pressure. This method is also time consuming and the accuracy is operator dependent.

There is therefore a need for a simple means of reliably investigating the condition of the ear of a subject that does not depend on the skill of the operator.

SUMMARY OF THE INVENTION

The invention is defined by the claims.

According to examples in accordance with an aspect of the invention, there is provided a system for assessing ear pathologies in a subject, the system comprising:

an earplug configured to be at least partially inserted in the ear canal of an ear of a subject,
wherein the earplug comprises a pressure sensor adapted to generate passive pressure signals representative of passive pressure changes within the ear canal induced by maneuvers performed by the subject,
wherein characteristics of the passive pressure signals are representative of one or more ear pathologies.

It is proposed that changes in pressure in the external ear canal may contain important information on ear pathologies, including pathologies in the external ear canal, in the middle ear and in the tympanic member (eardrum). The inventors have realized that an earplug can be used to isolate the external ear canal, such that the volume between the eardrum and the earplug becomes completely enclosed. In this way, the changes in pressure in the external ear canal (caused by pre-selected maneuvers) are dependent on the volume of the ear canal between earplug and eardrum, the movement and state of the eardrum, and presence or absence of pathologies. Thus, it is proposed that a pressure sensor in the earplug can be used to measure the changes in pressure in the ear canal and determine whether the changes correspond to a normal and healthy ear, or an ear with an ear pathology.

Thus, unlike conventional investigation approaches, such as tympanometry, which rely on the active application of (positive and/or negative) pressure to the ear canal and/or eardrum, proposed embodiments do not rely on complicated and/or intrusive equipment to apply pressure to the ear canal and/or eardrum. Instead, it is proposed to detect passive pressure changes in ear canal that are induced by maneuvers performed by the subject and, for this, a pressure sensor incorporated into an earplug is proposed.

Accordingly, it is to be understood that reference to active pressure changes is to be taken to refer to pressure changes that are caused by the active application of (positive and/or negative) pressure to a subject's ear canal by equipment or apparatus. Conversely, reference to passive pressure changes is to be taken to refer to pressure changes that naturally (or coincidently) result from movement and/or maneuvers performed by a subject, and thus do not require application of (positive and/or negative) pressure to a subject's ear canal by equipment or apparatus. Thus, there are proposed concepts for passive pressure detection, i.e. the detection of pressure changes within the ear canal through the passive use of a pressure sensors that simply detect pressure changes in the ear canal without controlled application of a (positive or negative) air pressure (i.e. non atmospheric pressure) to the ear canal.

For example, movement of the jaw causes the floor of the ear canal to move, thus changing the total enclosed volume between the earplug and the eardrum. By changing the volume in an enclosed space, the pressure inside the volume also passively changes. However, if the volume is not completely enclosed due to an ear pathology (e.g. eardrum perforation), the air in the external ear canal will be allowed to flow to the middle ear, and vice versa, and thus the changes in pressure will be different to the expected changes in pressure with no ear pathology. It is therefore proposed to sense such passive pressure changes that may be indicative of an ear pathology, and this sensing may be realized by the incorporation of a pressure sensor within an earplug (which can be situated in a subject's ear canal).

Proposed embodiments may comprise a relatively small and unobtrusive pressure sensing earplug that can obtain and supply information useful for investigating and/or identifying ear pathologies. Such embodiments may therefore be adapted to be continuously worn by a subject, because they do not require supplementary/additional equipment for applying a pressure to the subject's ear canal. Also, signals from the earplug may be communicated wirelessly to a remotely located processing unit for processing and/or investigation. In this way, continuous monitoring and/or non-obstructive investigation of a subject's ear pathologies may be facilitated.

In an embodiment, the system further comprises a processor configured to:

> receive the passive pressure signals from the pressure sensor of the earplug; and
> determine an indication of one or more ear pathologies in the ear of the subject based on the passive pressure signal, wherein characteristics of the passive pressure signals are further representative of the type of ear pathologies and the type of maneuver performed by the subj ect.

In this way, the system may be adapted to directly interpret the passive pressure signals obtained by way of the pressure sensor within the earplug to determine an indication of any ear pathologies present in the ear of the user.

In a further embodiment, the processor is further configured to receive an indication of the type of maneuver performed by the subject, and wherein determining an indication of ear pathologies is further based on the type of maneuver performed by the subject.

By further considering the type of maneuver performed by the user, the accuracy of the interpretation of the passive pressure signals is improved, thereby improving the accuracy of the determination of the indication of ear pathologies.

In a further embodiment, the system further comprises one or more of:

> a camera located within the earplug configured to image the ear canal;
> an external camera configured to monitor the maneuver performed by the subj ect;
> an accelerometer located within the earplug for determining the type of maneuver; and
> a gyroscope located within the earplug for determining the type of maneuver, or a combination thereof.

Various different sensors (e.g. camera, accelerometer and gyroscope) can be used to verify the maneuver performed by the subject and correlate the maneuver with physical changes or events occurring in the ear (e.g. tilting head, jaw movement etc.).

In an embodiment, the maneuver comprises actions performed by the subject resulting in passive pressure changes within the ear, optionally wherein the maneuver is one or more of:

moving jaw up and/or down;
moving jaw sideways;
swallowing;
yawning; and
tilting head,
or combination thereof.

Different maneuvers performed by the subject may allow different pathologies to be identified. Different maneuvers can also be performed by the same subject to provide a more accurate indication of the ear pathology.

For example, jaw movements up, down and/or sideways change the enclosed volume in the ear canal, thus the passive pressure changes in caused by these maneuvers contain information on the volume of the external ear canal as well as information on air flow within the ear. As mentioned, air flow within the external ear canal suggests a puncture in the eardrum. Additionally, abnormal volumes in the ear canal can suggest other pathologies, such as hard wax in the ear, retracted eardrum or bulging eardrum. A retracted or bulging eardrum suggests abnormal pressures in the middle ear (behind the eardrum), which in turn suggest an ear pathology in the middle ear.

Yawning and or swallowing provide an increase in pressure in the nasopharyngeal cavity. Alternatively, Valsalva's maneuver, Toynbee's maneuver, Frenzel's maneuver or drinking can be performed by the subject to increase the pressure in the nasopharyngeal cavity. The middle ear (behind the eardrum) is connected to the nasopharyngeal cavity by the Eustachian tube. Thus, abnormal passive pressure changes in the external ear canal may indicate a blocked Eustachian tube, as well as perforation in the eardrum, pathologies in the middle ear or hard wax in the external ear canal.

In an embodiment, determining an indication of an ear pathology further comprises:

determining the characteristics of the passive pressure signals, wherein the characteristics comprise one or more of:

amplitude values of the passive pressure signal;
peaks and/or valleys of the passive pressure signals;
temporal relationships of the peaks and/or valleys of the passive pressure signals;
a duration of the maneuver;
ratios of a subset of the passive pressure signals;
differences between peaks and valleys; and
frequency domain analysis, or a combination thereof, and

comparing the characteristics of the passive pressure signals to baseline signals wherein the baseline signals are representative of the type of maneuver performed by the subj ect.

By considering a variety of different characteristics of the passive pressure signals, it will be appreciated that the accuracy and flexibility of the system for arriving at a determination of the indication of an ear pathology may be improved. For example, by considering a wide variety of signal features, the most prominent features of a given signal may be selected in order to obtain an accurate comparison, which may then be used to obtain an accurate indication of an ear pathology.

In an embodiment, determining an indication of ear pathologies is further based on analyzing the passive pressure signals with a machine learning algorithm, wherein the machine learning algorithm has been trained to output an indication of ear pathologies.

By using a machine learning algorithm, such as one of the variety of known machine learning algorithms, the accuracy of the determination of the indication of the ear pathology may be improved. Further, the determination of the indication of the ear pathology may be performed with reduced input data.

In a further embodiment, the inputs of the machine learning algorithm are the characteristics of the passive pressure signals and one or more of:

maneuver type;
subject data, which comprises one or more of:

physical dimensions of the ear;
subject age;

subject gender;
a known subject condition
subject demographic data; and

a diagnosis of the subject.

In this way, additional information regarding the subject may be used to improve the accuracy of the determination of the ear pathology by considering additional factors that may affect the passive pressure changes in the ear of the subject.

In an embodiment, the earplug further comprises a humidity sensor for measuring the humidity inside the ear canal of the ear, wherein the humidity sensor is adapted to generate a humidity signal and wherein determining an indication of an ear pathology is further based on analyzing the humidity signal.

A humidity sensor can be used to measure the humidity in the external ear canal. Fluid discharge within the ear can increase humidity levels and typically indicate an unhealthy ear. Additionally, different fluids (serous, mucous, etc.) have different viscosities, thus different an ear pathology, corresponding to different fluids, can be indicated by the time taken for the humidity change to occur.

In an embodiment, the processor is further configured to:

receive a second passive pressure signal, wherein the second passive pressure signal is induced by a second maneuver performed by the subject, and wherein the processor is configured to determine an indication of one or more ear pathologies in the ear of the subject further based on characteristics of the second passive pressure signal, and optionally wherein the processor is adapted, when determining an indication of one or more ear pathologies in the ear of the subject further based on characteristics of the second passive pressure signal. to compare the characteristics of the passive pressure signal to the characteristics of the second passive pressure signal.

In this way, passive pressure changes within the ear of the subject may be monitored over a period of time, thereby providing additional information on the development of a potential ear pathology.

According to examples in accordance with an aspect of the invention, there is provided a method for assessing ear pathologies in a subject, the method comprising:

receiving passive pressure signals representative of passive pressure changes induced by maneuvers performed by the subject from a pressure sensor inserted in the ear canal of the subject; and
determining an indication of ear pathologies in the ear of the subject based on the passive pressure signal, wherein characteristics of the passive pressure signals are representative of one or more ear pathologies.

In an embodiment, the method further comprises receiving an indication of the type of maneuver performed by the subject, wherein determining an indication of ear pathologies is further based on the type of maneuver performed by the subject.

In an embodiment, determining an indication of ear pathologies comprises:

determining the characteristics of the passive pressure signals, wherein the characteristics comprise one or more of:

amplitude values of the passive pressure signals;
peaks and/or valleys of the passive pressure signals;
temporal relationships of the peaks and/or valleys of the passive pressure signals;
a duration of the maneuver;
ratios of a subset of the passive pressure signals;
differences between peaks and valleys of the passive pressure signals; and
frequency domain analysis, or combination thereof, and

comparing the characteristics of the passive pressure signals to baseline signals, wherein the baseline signals are representative of the type of maneuver performed by the subj ect.

In an embodiment, the method further comprises receiving a humidity signal corresponding to the humidity of the ear canal of the subject, wherein determining an indication of ear pathologies is further based on the humidity signal.

According to examples in accordance with an aspect of the invention, there is provided a computer program product comprising computer program code means which, when executed on a computing device having a processing system, cause the processing system to perform all of the steps of the method described above.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 shows a schematic representation of an ear of a subject;
Figure 2 shows a schematic representation of a system according to an aspect of the invention;
Figure 3 shows a graph of pressure against time for a plot of passive pressure when the subject performs the maneuver of opening and closing the jaw;
Figure 4 shows a graph of pressure against time for a plot of passive pressure when the subject performs the maneuver of yawning; and
Figure 5 shows a method of the invention.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for assessing ear pathologies in a subject. The system includes an earplug configured to be at least partially inserted in the ear canal of an ear of a subject. The earplug comprises a pressure sensor adapted to generate passive pressure signals representative of passive pressure changes within the ear canal induced by maneuvers performed by the subject, wherein characteristics of the passive pressure signals are representative of one or more ear pathologies.

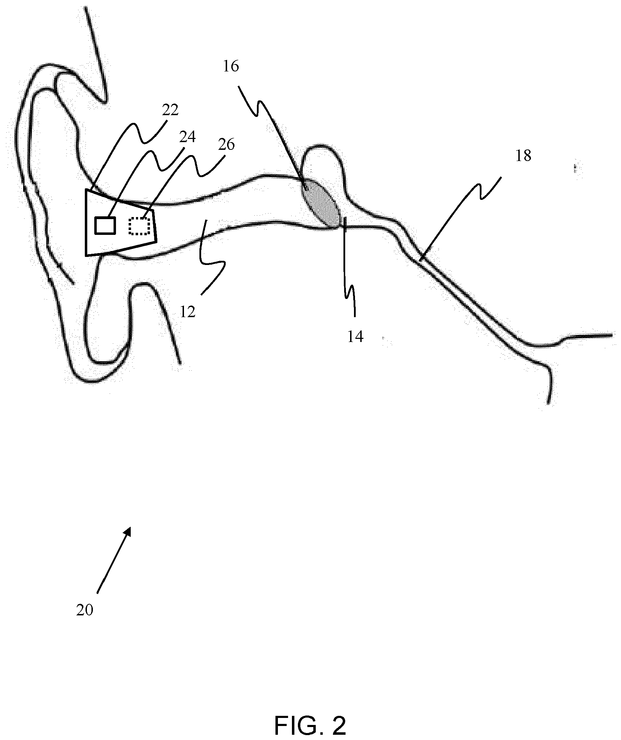

Figure 2 shows a system 20 for assessing ear pathologies in a subject.

In particular, Figure 2 shows an earplug 22 configured to be at least partially inserted in the ear canal, i.e. the exterior ear 12, of an ear of the subject. The earplug comprises a pressure sensor 24 adapted to generate passive pressure signals representative of passive pressure changes within the ear canal induced by maneuvers performed by the subject. The characteristics of the passive pressure signals are representative of one or more ear pathologies.

The term passive pressure signals and passive pressure changes refer to the changes in pressure within the ear of a subject that are not induced by any source external to the subject, but rather are induced by maneuvers performed by the subject, such as: moving the jaw up and/or down; moving the jaw sideways; swallowing; yawning; and tilting the head, or combination thereof.

Put another way, a passive pressure change occurs within the ear without requiring any application of positive or negative pressure (i.e. non-atmospheric pressure) by external apparatus or equipment and this change can be measured, which measurement then forms the passive pressure signal.

Pressure and humidity in the external ear canal contains important information regarding pathologies of the external ear, tympanic membrane and the middle ear; however, pressure changes in the external ear canal are not studied in as much detail as pressure changes in the middle ear. The pressure in the external ear 12 canal is closely linked with the status of tympanic membrane 16 and pressure in the middle ear 14, which in turn depends on a proper functioning of the Eustachian tube 18. The impact of perforation on pressure changes at tympanic membrane (eardrum) is well documented in literature. Pressure in the external ear canal has also been used to estimate changes in intracranial pressure as discussed in R. J. Marchbanks et al. "The effect of raised intracranial pressure on intracochlear fluid pressure: three case studies," Br J Audiol, vol. 21, no. 2, pp. 127-130, May 1987. However, no attempt has been made to use pressure changes in the external canal for diagnosis of different ear pathologies. This proposed systems and methods are based on passive introduction of pressure by subject movement, and optionally humidity, changes in the external ear canal by way of certain simple physical maneuvers performed by the subject, thereby eliminating the need for the application of external pressure to the ear canal. The pressure, and optionally humidity, changes may then be measured and analyzed to identify a potential pathology of the ear of the subject.

The proposed systems and methods are different from existing methods, such as pneumatic otoscopy or tympanometry as discussed above. The existing methods discussed above are based on studying the mobility of the tympanic membrane 16 and not on measuring the actual pressure in the external ear canal. As these methods rely on pressure changes in external ear induced by means of active pressure pumps, they introduce a risk of causing eardrum perforation.

The main elements of the system involve an earplug 22 integrated with a pressure sensors 24, and optionally with pressure sensors on both sides of the earplug. The earplug 22 may be used by a general physician (GP) for subject with a history of any ear complaints. The earplugs may inserted in both the ears either simultaneously or sequentially during the physical examination.

During examination, the subject is asked to perform certain simple maneuvers such as moving the jaw up and down; moving the jaw sidewise; swallowing; yawning; tilting head towards or away from the side of the earplug; and the like.

The earplug 22 may further include a humidity sensor 26 for measuring the humidity inside the ear canal of the ear, wherein the humidity sensor is adapted to generate a humidity signal.

The system may further comprises a processor configured to receive the passive pressure signals from the pressure sensor 24, and optionally the humidity signals from the humidity sensor 26, of the earplug 22. The processor may be any suitable processing unit in wired or wireless communication with the sensors of the earplug.

The processor is further adapted to determine an indication of one or more ear pathologies in the ear of the subject based on the passive pressure signal, and optionally further based on the humidity signal, wherein characteristics of the passive pressure signals are further representative of the type of ear pathologies and the type of maneuver performed by the subject.

The term indication may refer to the presence or absence of a given ear pathology.

For example, determining an indication of an ear pathology may include determining the characteristics of the passive pressure signals, such as: amplitude values of the passive pressure signal; peaks and/or valleys of the passive pressure signals; temporal relationships of the peaks and/or valleys of the passive pressure signals; duration of the maneuver; ratios of a subset of the passive pressure signals; differences between peaks and valleys; and frequency domain analysis, or a combination thereof.

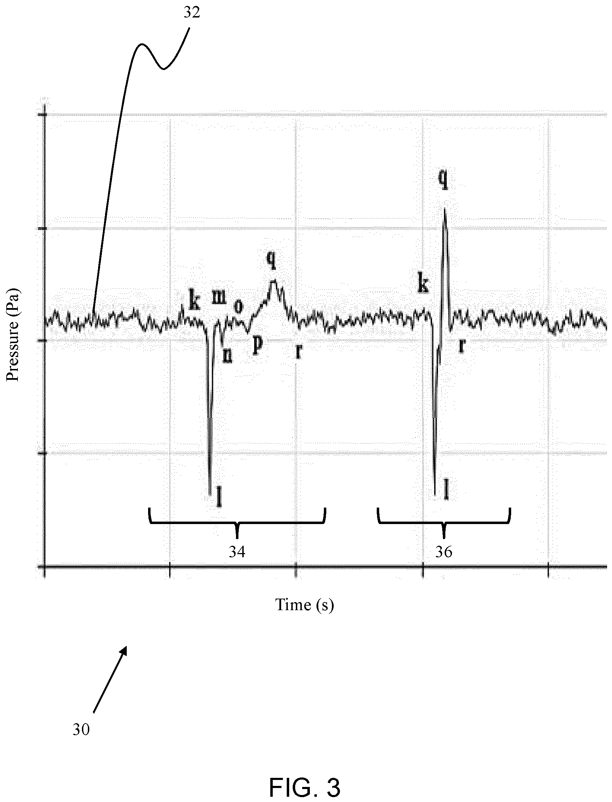

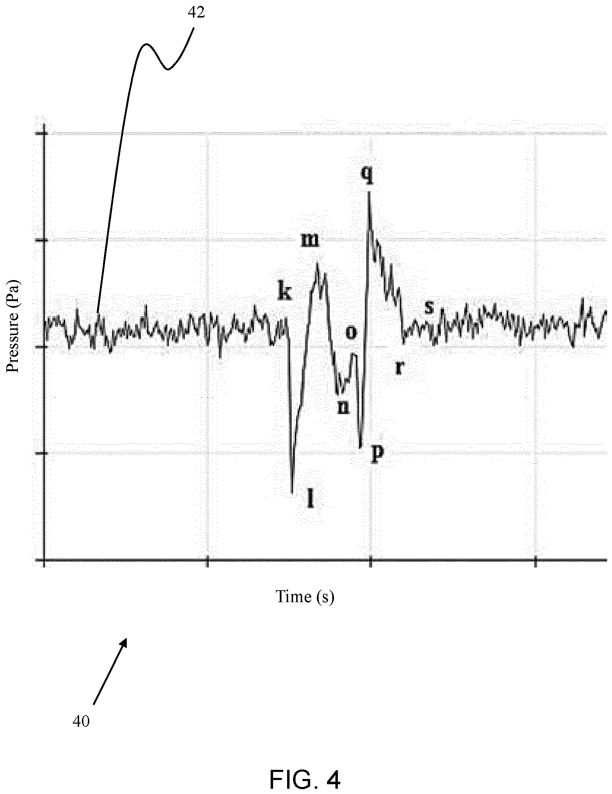

The characteristics of the passive pressure signals may be compared to baseline signals, wherein the baseline signals are representative of the type of maneuver performed by the subject. Specific examples and implementations of how the passive pressure signals may be used to derive the ear pathologies are described below with reference to Figures 3 and 4.

Alternatively, or in addition, to the baseline signals, the passive pressure signals may be compared to previously acquired passive pressure signals. The previously acquired passive pressure signals may have been acquired during a previous ear examination, or during the same ear examination. The previously acquired passive pressure signals may have resulted from the same type of maneuver as the subsequent passive pressure signals.

Put another way, the processor may be adapted to receive a second passive pressure signal, wherein the second passive pressure signal is induced by a second maneuver performed by the subject. The processor may then be configured to determine an indication of one or more ear pathologies in the ear of the subject further based on characteristics of the second passive pressure signal. For example, the processor may be adapted, when determining an indication of one or more ear pathologies in the ear of the subject further based on characteristics of the second passive pressure signal, to compare the characteristics of the passive pressure signal, which may have been acquired previously, to the characteristics of the second passive pressure signal.

Further, a plurality of successive passive pressure signals measurements may be used to build a passive frequency trend in order to monitor a change in the ear pathology.

In addition to the passive pressure signals, and optionally the humidity signals, the processor may be further configured to receive an indication of the type of maneuver performed by the subject, which results in a corresponding change in passive pressure, in which case determining the indication of ear pathologies of the ear of the subject is further based on the type of maneuver performed by the subject.

Put another way, as known changes in passive pressure within the ear may be linked with the type of maneuver performed by the subject, by identifying the type of maneuver performed by the subject when obtaining passive pressure signals, the accuracy the determination of the indication of the ear pathology may be increased.

The identification of the type of maneuver may be performed by any means suitable of monitoring a movement of the subject. For example, the system may include: a camera located within the earplug configured to image the ear canal, wherein the maneuver may be identified based on how the ear canal moves when the maneuver is performed; an external camera configured to monitor the maneuver performed by the subject, such as a camera positioned at a given point in a room occupied by the subject; an accelerometer located within the earplug, or placed externally on the jaw of the subject, for determining the type of maneuver, for example by monitoring a motion of the subject's head; a gyroscope located within the earplug for determining the type of maneuver; or a combination thereof.

The information from the different sensors is then correlated to analyze change in the external ear cavity pressure, and optionally humidity, when the different maneuvers are performed.

Determining an indication of ear pathologies may be performed by analyzing the passive pressure signals with a machine learning algorithm, wherein the machine learning algorithm has been trained to output an indication of an ear pathology.

A machine-learning algorithm is any self-training algorithm that processes input data in order to produce or predict output data. Here, the input data may comprise the characteristics of the passive pressure signals and one or more of: a maneuver type, duration, strength, speed; subject data, such as physical dimensions of the ear, subject age, subject gender, a known subject condition and subject demographic data; and a diagnosis of the subject and the output data comprises an indication of an ear pathology. The physical dimensions of the ear may include, for example, dimensions of the ear canal, ear drum and the like.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. $\pm 1\%$) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries correspond to example pressure signal characteristics. The training output data entries correspond to an example indication of an ear pathology.

The following description provides examples of the systems and methods according to aspects of the invention when in use.

Whilst a subject ear examination may be carried out with a single ear plug applied successively to the ears or with two ear plugs applied simultaneously to both ears, the description herein relates to the case of using two ear plugs applied simultaneously to the ears of the subject.

Earplugs, such as the earplug 22 shown in figure 2, are inserted in the external ear 12 such that they seal the ear canal from the external atmosphere, thereby creating a closed chamber with the pressure senor 24 on one side of the closed chamber and the tympanic membrane 16 on other side.

Initially, the subject is asked to close their mouth, in such a way that they lock the jaws, and the pressure on both sides of the earplug are measured, i.e. the atmospheric pressure outside the ear and the pressure within the ear canal are measured.

The subject may then be asked to perform one or more of the maneuverers as follows for a systematic ear examination.

The first maneuver may comprise upward and downward jaw movements. For example, the subject may be asked to move the lower jaw down to the fullest possible extent and then move it up so as to close the mouth. The jaw movements happen at the temporomandibular joints, which are the two joints connecting the mandible (jawbone) to the skull. These joints are located anterior and below the floor of external ear canal, which is cartilaginous. The downward movement of the jaw creates a depression in the floor of external ear canal, thereby effectively increasing the volume of the closed chamber formed by earplug 22, comprising the pressure senor 24, and the tympanic membrane 16.

As defined by Boyles' law, the absolute pressure (P) exerted by a given mass of an ideal gas is inversely proportional to the volume (V) it occupies if the temperature and amount of gas remain unchanged within a closed system. This can be expressed mathematically as,

$$P \propto \left(\frac{1}{V}\right)$$

The external ear canal is a roughly elliptical cylinder and is typically 2.5 cm long with a diameter of 0.7 cm. To measure the passive pressure changes, a lossless cylindrical-tube model may be used as an approximation for the extremal ear canal as described in S. E. Voss et al. "Middle-ear function with tympanic-membrane perforations. I. Measurements and mechanisms," J. Acoust. Soc. Am., vol. 110, no. 3 Pt 1, pp. 1432-1444, Sep. 2001. The volume of the closed chamber created by the earplugs (V) may be calculated as,

$$V = \pi r^2 h,$$

where, *h* is the distance of the pressure sensor from the tympanic membrane and r is the radius of the closed chamber.

For the calculation of the volume, subject data such as typical age and gender specific values for *h* may be used or exact values for the individual subject may be calculated using suitable sensors, such as proximity sensors, ultrasound sensor, computed tomography, and the like. Similarly, normal age and gender specific values for the diameter of the ear canal may be used for the calculation or exact values may be obtained as described above.

As the downward movement of the jaw increases the volume of the closed chamber formed by the earplug and the tympanic membrane, this maneuver results in a decrease in the passive pressure within the external ear cavity.

Figure 3 shows a graph 30 of pressure against time, wherein plot 32 represents a passive pressure signal measure within the external ear canal of a subject by way of the earplug pressure sensor described above with respect to Figure 2.

More specifically, the plot 32 of Figure 3, shows the change in pressure in the external ear cavity during downward and upward movements of the jaw of the subject. The first half of the plot 34 shows a jaw drop followed by a delayed closing of the jaw; the second half of the plot 36 shows a rapid down and up movement of the jaw. The baseline pressure in the external ear canal in denoted by *k*. The sudden opening of the jaw results in a sharp decrease in pressure (represented by valley '*l*' on the plot 32). The magnitude of the pressure drop may be calculated as follows,

$$\text{Pressure drop jaw (PDJ)} = |k - l|$$

The pressure drop is followed by a slow increase in pressure due to seepage of air from the external atmosphere into the chamber between the earplug and tympanic membrane (as shown in the plot by the segment between points *l* and *m*) from the small gap between the bottom of the earplug and the depression in the floor of the external ear canal. The closing of the jaw initially results in a slight drop in pressure (as shown in the plot by the segment between points *o* and *p*) followed by a rapid increase in pressure (as shown in the plot by the segment between points *p* and *q*) in the external ear canal. The magnitude of the increase in pressure may be calculated as follows,

$$\text{Pressure increase jaw (PIJ)} = |p - q|$$

The pressure then gradually stabilizes to the baseline pressure (at point *r* on the plot).

During fast movements of the jaw, only a rapid decrease and increase in pressure is clearly visible with the intermediate steps being significantly truncated. It is important to note that the exact pressure changes in the external ear canal depends on many factors such as the dimensions of the ear canal, the distance between the pressure sensor and the tympanic membrane, the state of the tympanic membrane, the speed of the jaw movements, the magnitude of the movements, the middle ear pressure, and the like.

The total passive pressure change during an up and down movement of the jaw may be calculated as follows,

$$\text{Total pressure change jaw (TPCJ)} = \text{PDJ} + \text{PIJ}$$

Measurements from a sufficiently large sample population may be obtained and use to create a database of expected changes for PDJ, PIJ, TPCJ or baseline characteristics of pressure changes associated with a given maneuver. A given subject may be asked to perform the same set of movements repeatedly in order to obtain average passive pressure signal characteristics for deriving an indication of the ear pathology. If a subject is suspected of having an ear pathology in one ear with the other ear being health, the signal from the healthy ear may be used as a reference signal.

Different pathological conditions are likely to have different impacts on the characteristics of the passive pressure changes leading to changes in the characteristics of the obtained passive pressure signals. This information can be used to derive an indication of the presence, or absence, of different pathologies as follows.

For example, a subject may have an ear pathology comprising a perforation in the tympanic membrane (eardrum).

A perforation in the tympanic membrane acts as a channel between the external ear cavity and the middle ear cavity, meaning the closed chamber created by the earplug will have an additional opening at the tympanic membrane end.

In this case, the downward movement of the jaw is likely to result in a momentary decrease in pressure followed by a rapid increase in pressure due to the influx of air from the middle ear cavity via the perforation in the tympanic membrane. Therefore, the pressure drop PDJ in the case where there is a perforation in the tympanic membrane will have a reduced magnitude compared to a normal population, or a heathy ear.

In addition, due to the rapid increase in pressure resulting from the influx of air, the time taken to reach to the baseline pressure (i.e. the time duration between points *l* and *m* on the plot 32) will be shorter in this case.

Similarly, the pressure increase PIJ in the case of perforation will have a reduced magnitude compared to a normal population or a healthy ear. Therefore, the TPCJ in the case of perforation will also have a reduced magnitude compared to that of an ear without any tympanic membrane perforation. In general, the magnitude of TPCJ is inversely proportional to the size of the perforation, with a larger perforation leading to a lower TPCJ.

TPCJ values obtained from a sample population may be used to derive certain thresholds that are indicative of the presence of a perforation and the size of said perforation. As such, differential measurements of the two ears (assuming one ear to be healthy) will indicate if there is a perforation of one of the membranes. Alternatively, a machine learning model trained on a database of cases with varying degrees of perforation size may be used to analyze the magnitude of pressure changes to detect and quantify a perforation in the tympanic membrane.

In a further example, a subject may have a pathology in their middle ear.

The pressure in the middle ear is maintained very close to atmospheric pressure; however, it may become negative or positive (with respect to atmospheric pressure) as a result of certain pathological conditions. There is a known association between negative middle ear pressure and middle ear pathologies with middle ear pressure observed in the range of -1000 to -4000 Pa by tympanometry in such cases as discussed in M. Gaihede, "Middle ear volume and pressure effects on tympanometric middle ear pressure determination: model experiments with special reference to secretory otitis media," Auris Nasus Larynx, vol. 27, no. 3, pp. 231-239, Jul. 2000. On the other hand, a collapsed Eustachian tube and fluid collection in the middle ear may be associated with a transient positive pressure.

A change in the middle ear pressure results in tympanic membrane displacement (bulging for positive pressure or retraction for negative pressure). There is a non-linear relationship between ear canal pressure and tympanic membrane volume displacement. The tympanic membrane volume displacement is in the range of $+ 20$ mm$^3$ with the corresponding ear canal pressure changes in the range of $- 1.8$ to $2.4$ kPa as discussed in M. Gaihede, "Middle ear volume and pressure effects on tympanometric middle ear pressure determination: model experiments with special reference to secretory otitis media," Auris Nasus Larynx, vol. 27, no. 3, pp. 231-239, Jul. 2000.

Negative pressure in the middle ear results in a retracted tympanic membrane. Due to the retracted tympanic membrane, the closed chamber created by the pressure sensor in the earplug and the tympanic membrane will have a higher volume compared to a healthy ear. Therefore, the pressure decrease during jaw drop (PDJ) will have a reduced magnitude compared to a healthy ear; whereas, in the case of positive middle ear pressure, the drop will have a higher magnitude.

Similarly to up and down movements of the jaw, the subject may also perform sidewise jaw movements. During this movement the mandible condyle of the same side moves down and outwards, thereby creating a lateral and downward depression in the floor of the external cavity. Similarly to the up and down movement of the jaw, the resultant change in passive pressure is measured by the earplug and the difference in pressure during these movements may then be used to find the different pathologies.

An alternative, or subsequent, maneuver to be performed by the subject may comprises yawning or swallowing. The subject may be asked to swallow, yawn or perform a similar maneuver, such as a Valsalva maneuver, Toynbee maneuver, Frenzel maneuver or drinking that increases the pressure in the naso-pharyngeal cavity. The middle ear is connected to the nasopharynx via the Eustachian tube. Accordingly, any of these maneuvers may be used to increase the pressure in the middle ear cavity. A change in the middle ear pressure results in tympanic membrane displacement (such as bulging or retraction).

An increase in the middle pressure during yawning results in a transient bulging of the tympanic membrane toward the external ear canal. The earplug is used to measure the corresponding passive pressure change in the external ear canal during these maneuverers. The difference in pressure during these maneuvers may then be used to find different pathologies.

Yawning consists of opening the mouth wide (a maximal widening of the jaw), together with a long and deep inhalation through the mouth and nose, followed by a slow expiration and finally the closing of the mouth.

Figure 4 shows a graph 40 of pressure against time, wherein plot 42 represents a passive pressure signal measure within the external ear canal of a subject by way of the earplug pressure sensor described above with respect to Figure 2.

More specifically, the plot 42 of Figure 4 shows a passive pressure signal representative of the passive pressure changes in the external ear during a yawning maneuver. The baseline pressure in the external ear canal in denoted by *k*. The segment between points *k* and *l* denotes a pressure drop in the external ear due to the jaw opening in the initial stage of yawning. The pressure drop due to downward movement of the jaw may be calculated as,

$$\text{Pressure drop jaw (PDJ)} = |k - l|$$

During yawning, the chief route of air entry is through the mouth, meaning there is little orthogonal air flow past the Eustachian tube openings in the nasopharynx, which keeps the reduction of pressure due to the Bernoulli effect minimal. This is followed by increase in pressure (shown by the segment between points *l* and *m*) due to the transmission of a positive pressure built in the middle ear through the Eustachian tube during deep inhalation to the external ear canal via bulging of the tympanic membrane. As the Eustachian tube opens, the pressure inside the Eustachian tube and the middle ear starts equilibrating quickly with the nasopharynx pressure (shown by the early part of the *l* to *m* phase), the rate of which decreases before suddenly accelerating. This acceleration may be due to the backflow of air from the oropharynx to the nasopharynx. The pressure increase due to yawning may be calculated as follows,

$$\text{Pressure increase yawning (PIY)} = |k - m|$$

A slow expiration following the inhalation leads to a slight drop in pressure in the middle ear (as shown by segment *m* to *n*) followed by a recovery of the pressure (as shown by segment *n* to *o*). The pressure drop following the yawn may be calculated as,

$$\text{Pressure drop yawning (PDY)} = |m - n|$$

The snap closing of jaw at the end of the yawn results in a slight drop in pressure (as shown by segment *o* to *p*) followed by a rapid increase in pressure in the external ear (as shown by segment *p* to *q*). The pressure then gradually returns to the baseline pressure at point *s*. The increase in pressure due to the upward movement of the jaw may be calculated as,

$$\text{Pressure increase jaw (PIJ)} = |p - q|$$

The total passive pressure change during the up and down movement of jaw during the yawn maneuver may be calculated as,

$$\text{Total pressure change jaw (TPCJ)} = \text{PDJ} + \text{PIJ}$$

The total passive pressure change during yawning may be calculated as,

$$\text{Total pressure change jaw (TPCY)} = \text{PIY} + \text{PDY}$$

The ratio of passive pressure change due to the jaw moving downward to the inhalation during the initial stage of yawning may be expressed as follows,

$$\text{Ratio of pressure change (RPC)} = \text{PDJ/PIY}$$

The ratio of PDJ to PIY provides important information regarding the status of the tympanic membrane and middle ear pressure. The RPC is typically > 2; however, this ratio depends upon many parameters such as patency of the Eustachian tube, speed and magnitude of jaw movement, presence of wax in the external ear and the like. Values of different pressure changes such as PDJ, PIY, and RPC obtained from a population may be used to derive certain thresholds, which are indicative of different ear pathologies.

Different pathological conditions are likely to have different impacts on the characteristics of pressure changes. This information can be used to detect different pathologies as follows.

One such pathology may be a perforation in the tympanic membrane with a functional Eustachian tube. A perforation in the tympanic membrane acts as a channel between the external ear cavity and the middle ear cavity as described above.

In the case of perforation, the magnitude of PDJ will be reduced and the magnitude of PIY will be increase. Therefore, RPC is likely to be < 2 in the case of a large perforation. The magnitude of the passive pressure change in this case will depend upon the diameter of the perforation. Population-based threshold values of PDJ and PIY may be used to diagnose the perforation and its size. In additional, differential measurements of the two ears may also be used to indicate if there is a perforation of one of the membranes. Alternatively, a model trained on a database of cases with perforations of different sizes may be used to quantify a perforation size.

Another such pathology may be a perforation in the tympanic membrane with a blocked Eustachian tube.

The Eustachian tube connects the middle ear cavity with the nasopharynx. When there is a blockage of the Eustachian tube, there will be no pressure change in the middle ear during yawning. The downward movement of the jaw in this case, is likely to result in a momentarily decrease in pressure followed by a rapid increase in pressure due to influx of air from the middle ear cavity via the perforation.

In the case of a perforation of the tympanic membrane with a blocked Eustachian tube, the magnitude of the PDJ will be reduced. The magnitudes of both the PIY and the PDY will be reduced. The magnitude of the passive pressure change in this case will depend upon the diameter of the perforation and the extent of the Eustachian tube blockage. As such, differential measurements between the two ears of the subject will indicate if there is a perforation of one of the membranes. Alternatively, a model trained on a database of cases with perforations of different sizes and blocked Eustachian tubes may be used to quantify the perforation size.

The negative pressure in the middle ear caused by the blocked Eustachian tube will result in a retracted tympanic membrane. Due to the retracted tympanum membrane, the chamber created by the earplug, comprising the pressure senor, and the tympanic membrane will have a higher volume when compared to a heathy ear. Therefore, the magnitude of the pressure during jaw drop (PDJ) will be reduced compared to a healthy ear. Both the PIY and the PDY will be absent, or reduced. In the alternate case of positive middle ear pressure, the magnitude of the PDJ will be higher with an absent or reduced PIY and PDY.

A further example of a potential ear pathology is a collection of hard wax in the external ear. The wax collected in the external ear can partially, or completely, block the exterior ear canal. In such cases, the presence of the wax between the pressure sensor and tympanic membrane will prevent the transmission of pressure changes from the middle ear to the external ear. Therefore, the '*m*' peak shown in Figures 3 and 4 would be either absent or of a reduced magnitude compared to normal circumstances in the absence of wax. Similarly, a slow drop in pressure (shown by the segments between *m* and *n*) due to exhalation will be shorter, or absent. A similar effect may be also expected in the case of a very stiff tympanic membrane.

The impact of hard wax on the PDJ and the PIJ will depend upon the extent of the wax in the ear canal, which affects the distance between the wax and the pressure sensor. Both the PIY and the PDY will have a reduced magnitude, or be absent entirely. As such, differential measurements between the two ears of the subject will indicate if there is wax collected in the external ear canal of one or other of the ears. Alternatively, a model trained on a database of cases with a varying degree of wax may be used to analyze the magnitude of pressure changes to quantify the amount of wax in the external ear.

Table 1: A table showing typical relative pressure changes associated with a given maneuver when a given pathology is present in the ear when compared to a baseline pressure

| Pathology | PDY | PU | TPCI | PIV | PDY | TPCY | RPC |
|---|---|---|---|---|---|---|---|
| Normal | Baseline | Baseline | Baseline | Baseline | Baseline | Baseline | Baseline |
| PTM | ↓↓ | ↓↓ | ↓↓ | ↑↑↑ | ↓↓ | ↑↑↑ | ↓↓↓ |
| PTM+BET | ↓↓ | ↓↓ | ↓↓ | ↓↓↓/x | ↓↓/x | ↓↓↓/x | ↑/x |
| Negative MEP | ↓ | ↓ | ↓ | ↓↓↓/x | ↓↓/x | ↓↓↓/x | ↑↑/x |
| Positive MEP | ↑ | ↑ | ↑ | ↓↓↓/x | ↓↓/x | ↓↓↓/x | ↑↑↑/x |
| Hard wax | | | | x | x | x | |

In table 1 above: BET represents a blocked Eustachian tube; MEP represents middle mar pressure; PTM respresents a perforated Tympanic membrane; **X** represents an absence of any pressure change; an upward arrow indicates an increase in pressure; a downward arrow indicates a decrease in presusre; and the number of arrows represents the mangitude of the pressure change.

In addition to the passive pressure signals described above, the earplug may further include a humidity senor to detect the presence of fluid in the external ear canal along with the different pathologies as described above. Fluid discharge in the ear canal is associated with a high relative humidity in the ear canal. If the relative humidity in the ear canal is significantly higher than the relative humidity outside of the ear, such as more than 30%, or if relative humidity in the ear canal is more than 70%, then it indicates an ear pathology with some discharge as discussed in S. E. Voss, J. J. Rosowski, S. N. Merchant, and W. T. Peake, "Middle-ear function with tympanic-membrane perforations. I. Measurements and mechanisms," J. Acoust. Soc. Am., vol. 110, no. 3 Pt 1, pp. 1432-1444, Sep. 2001.

In this case, the earplugs are inserted in the external ear canal such that they seal the ear cavity from the external atmosphere. The subject is then asked to perform physical maneuverers as described above. In addition to the maneuvers described above, the subject may be asked to perform additional maneuvers as follows for a systematic humidity based examination.

For example, the subject may be asked to tilt their head towards and away from the ear with the earplug inserted, thereby causing the fluid, if any, to gravitate towards the ear canal. The change in pressure and humidity may then be measured using the pressure and humidity sensors and used to obtain an indication of different ear pathologies in addition to an indication of the presence or absence of fluid in the ear canal.

As the different types of fluids that occur in ears, such as serous fluid, mucous and the like, are known to have different viscosity and elasticity properties as detailed in K. Takeuchi et al. "Viscoelastic properties of middle ear effusions from pediatric otitis media with effusion and their relation to gross appearance," Eur Arch Otorhinolaryngol, vol. 247, no. 1, pp. 60-62, Jan. 1990, the time taken for a change in pressure or humidity to occur may also be used to categorize fluid type.

In addition to the tilting of the head the subject may simultaneously perform a swallowing or yawning maneuver that results in an increase in the middle ear pressure with air travelling from the nasal cavity via the Eustachian tube. The swallowing or yawning may be used as an additional force to help the fluid to gravitate towards to the ear canal. The difference in pressure during these positions may then be used to identify an ear pathology.

Sudden changes in the pressure across the tympanic membrane, referred to as Barotrauma, may lead to blocked ears or traumatic perforations of the tympanic membrane. Such incidences are common during activities such as air travel, explosions or a sudden blast, weight lifting, scuba divining and the like. In such cases, the continuous monitoring of passive pressure in the external ear canal may be used to measure passive pressure changes over time. Using earlier measurements as a reference or baseline, the pressure changes may be used to diagnose ear pathologies as described above.

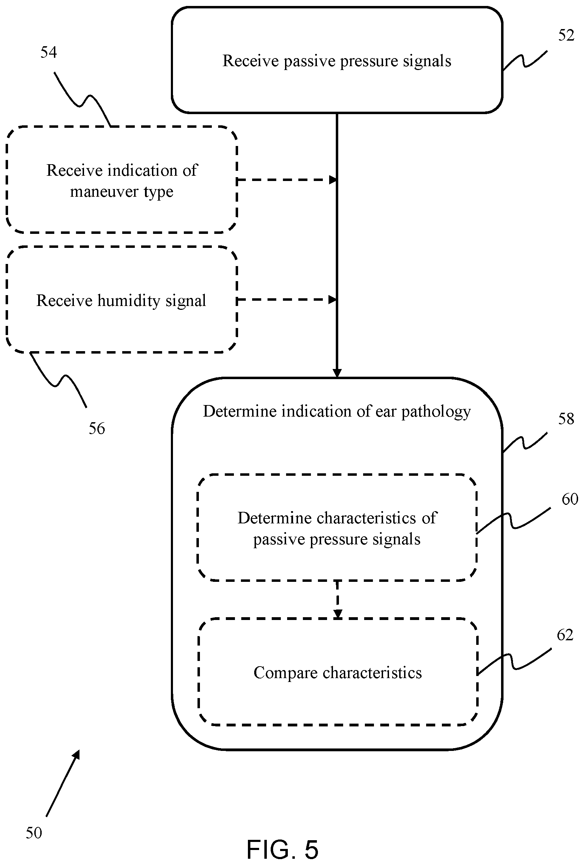

Figure 5 shows a method 50 for assessing ear pathologies in a subject.

The method begins in step 52 by receiving passive pressure signals representative of passive pressure changes induced by maneuvers performed by the subject from a pressure sensor inserted in the ear canal of the subject. Examples of the maneuvers that may be performed by the subject are described above.

Optionally, the method may further comprise the step of receiving 54 an indication of the type of maneuver performed by the subject, wherein determining an indication of ear pathologies is further based on the type of maneuver performed by the subject.

Further, the method may additional comprise the step of receiving 56 a humidity signal corresponding to the humidity of the ear canal of the subject, wherein determining an indication of ear pathologies is further based on the humidity signal.

In step 58, an indication of ear pathologies in the ear of the subject is determined based on the passive pressure signal, wherein characteristics of the passive pressure signals are representative of one or more ear pathologies.

Determining the indication of the ear pathologies may include the step of determining 60 the characteristics of the passive pressure signals, wherein the characteristics may include: an amplitude value of the passive pressure signals; a peak and/or valley of the passive pressure signals; a temporal relationships of the peaks and/or valleys of the passive pressure signals; a duration of the maneuver; a ratio of a subset of the passive pressure signals; and differences between peaks and valleys of the passive pressure signals, frequency domain analysis, or a combination thereof. Further, the step of determining the indication of the ear pathologies may include the step of comparing 62 the characteristics of the passive pressure signals to baseline signals, wherein the baseline signals are representative of the type of maneuver performed by the subject.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

**Claims**

1. A system (20) for assessing ear pathologies in a subject, the system comprising:

   an earplug (22) configured to be at least partially inserted in the ear canal of an ear of a subject, wherein the earplug comprises a pressure sensor (24) adapted to generate passive pressure signals representative of passive pressure changes within the ear canal induced by maneuvers performed by the subject, wherein characteristics of the passive pressure signals are representative of one or more ear pathologies.

2. The system (20) of claim 1, further comprising a processor configured to:

   receive the passive pressure signals from the pressure sensor of the earplug; and
   determine an indication of one or more ear pathologies in the ear of the subject based on the passive pressure signal, wherein characteristics of the passive pressure signals are further representative of the type of ear pathologies and the type of maneuver performed by the subj ect.

3. The system (20) of claim 2, wherein the processor is further configured to receive an indication of the type of maneuver performed by the subject, and wherein determining an indication of ear pathologies is further based on the type of maneuver performed by the subject.

4. The system (20) of claim 3, wherein the system further comprises one or more of:

   a camera located within the earplug configured to image the ear canal;
   an external camera configured to monitor the maneuver performed by the subj ect;
   an accelerometer located within the earplug for determining the type of maneuver; and
   a gyroscope located within the earplug for determining the type of maneuver, or a combination thereof.

5. The system (20) of any one of claims 1 to 4, wherein the maneuver comprises actions performed by the subject resulting in passive pressure changes within the ear, optionally wherein the maneuver is one or more of:

   moving jaw up and/or down;
   moving jaw sideways;
   swallowing;
   yawning; and
   tilting head,
   or combination thereof.

6. The system (20) of any one of claims 2 to 5, wherein determining an indication of an ear pathology further comprises:

   determining the characteristics of the passive pressure signals, wherein the characteristics comprise one or more of:

   amplitude values of the passive pressure signal;
   peaks and/or valleys of the passive pressure signals;
   temporal relationships of the peaks and/or valleys of the passive pressure signals;
   a duration of the maneuver;
   ratios of a subset of the passive pressure signals;
   differences between peaks and valleys; and
   frequency domain analysis, or a combination thereof, and

   comparing the characteristics of the passive pressure signals to baseline signals, wherein the baseline signals are representative of the type of maneuver performed by the subj ect.

7. The system (20) of any one of claims 2 to 6, wherein determining an indication of ear pathologies is further based on analyzing the passive pressure signals with a machine learning algorithm, wherein the machine learning algorithm has been trained to output an indication of ear pathologies.

8. The system (20) of claim 7, wherein the inputs of the machine learning algorithm are the characteristics of the passive pressure signals and one or more of:

maneuver type;
subject data, which comprises one or more of:

physical dimensions of the ear;
subject age;
subject gender;
a known subject condition
subject demographic data; and

a diagnosis of the subject.

9.  The system (20) of any one of claims 2 to 8, wherein the earplug further comprises a humidity sensor (26) for measuring the humidity inside the ear canal of the ear, wherein the humidity sensor is adapted to generate a humidity signal and wherein determining an indication of an ear pathology is further based on analyzing the humidity signal.

10. The system (20) of any one of claims 2 to 9, wherein the processor is further configured to:
    receive a second passive pressure signal, wherein the second passive pressure signal is induced by a second maneuver performed by the subject, and wherein the processor is configured to determine an indication of one or more ear pathologies in the ear of the subject further based on characteristics of the second passive pressure signal, and optionally wherein the processor is adapted, when determining an indication of one or more ear pathologies in the ear of the subject further based on characteristics of the second passive pressure signal. to compare the characteristics of the passive pressure signal to the characteristics of the second passive pressure signal.

11. A method (50) for assessing ear pathologies in a subject, the method comprising:

receiving (52) passive pressure signals representative of passive pressure changes induced by maneuvers performed by the subject from a pressure sensor inserted in the ear canal of the subject; and
determining (58) an indication of ear pathologies in the ear of the subject based on the passive pressure signal, wherein characteristics of the passive pressure signals are representative of one or more ear pathologies.

12. The method (50) of claim 11, further comprising receiving (54) an indication of the type of maneuver performed by the subject, wherein determining an indication of ear pathologies is further based on the type of maneuver performed by the subject.

13. The method (50) of any one claims 11 or 12, wherein determining (58) an indication of ear pathologies comprises:

determining (60) the characteristics of the passive pressure signals, wherein the characteristics comprise one or more of:

amplitude values of the passive pressure signals;
peaks and/or valleys of the passive pressure signals;
temporal relationships of the peaks and/or valleys of the passive pressure signals;
a duration of the maneuver;
ratios of a subset of the passive pressure signals;
differences between peaks and valleys of the passive pressure signals; and
frequency domain analysis, or combination thereof, and

comparing (62) the characteristics of the passive pressure signals to baseline signals, wherein the baseline signals are representative of the type of maneuver performed by the subject.

14. The method (50) of any one of claims 10 to 13, further comprising receiving (56) a humidity signal corresponding to the humidity of the ear canal of the subject, wherein determining an indication of ear pathologies is further based on the humidity signal.

15. A computer program product comprising computer program code means which, when executed on a computing device having a processing system, cause the processing system to perform all of the steps of the method according to any of claims 11 to 14.

16

18

12

14

10

FIG. 1

FIG. 2

FIG. 3

FIG. 4

FIG. 5

Europäisches
Patentamt

European
Patent Office

Office européen
des brevets

# EUROPEAN SEARCH REPORT

**Application Number**

EP 20 18 5050

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2007/112290 A1 (MORITA MASAHIRO [JP]) 17 May 2007 (2007-05-17) * paragraphs [0095] - [0099]; figures 5,6 * | 1-15 | INV. A61B5/11 A61B5/12 G16H50/20 |
| X | CN 110 584 609 A (BEIJING ZHUOCHI TECH CO LTD) 20 December 2019 (2019-12-20) * sections "Background technique", "Example one"; figures 1-3 * | 1-15 | |
| X | JP H10 127670 A (MATSUMOTO TOSHIHIKO; NAGASHIMA MEDICAL INSTR) 19 May 1998 (1998-05-19) * paragraphs [0009] - [0031]; figures 1-6 * | 1-15 | |
| A | US 2002/143242 A1 (NEMIROVSKI GUERMAN G [US]) 3 October 2002 (2002-10-03) * paragraphs [0047] - [0082]; figures 1-5 * | 1-15 | |
| A | US 2018/220904 A1 (LEBOEUF STEVEN FRANCIS [US] ET AL) 9 August 2018 (2018-08-09) * paragraphs [0066] - [0151]; figures 1-19 * | 1-15 | TECHNICAL FIELDS SEARCHED (IPC) A61B |

The present search report has been drawn up for all claims

| Place of search | Date of completion of the search | Examiner |
|---|---|---|
| The Hague | 10 December 2020 | Mecking, Nikolai |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 18 5050

This annex lists the patent family members relating to the patent documents cited in the above-mentioned European search report.
The members are as contained in the European Patent Office EDP file on
The European Patent Office is in no way liable for these particulars which are merely given for the purpose of information.

10-12-2020

| Patent document cited in search report | | Publication date | Patent family member(s) | | Publication date |
|---|---|---|---|---|---|
| US 2007112290 | A1 | 17-05-2007 | JP | 4597864 B2 | 15-12-2010 |
| | | | JP | WO2005044161 A1 | 17-05-2007 |
| | | | US | 2007112290 A1 | 17-05-2007 |
| | | | WO | 2005044161 A1 | 19-05-2005 |
| CN 110584609 | A | 20-12-2019 | NONE | | |
| JP H10127670 | A | 19-05-1998 | JP | 3810026 B2 | 16-08-2006 |
| | | | JP | H10127670 A | 19-05-1998 |
| US 2002143242 | A1 | 03-10-2002 | NONE | | |
| US 2018220904 | A1 | 09-08-2018 | EP | 2094152 A1 | 02-09-2009 |
| | | | EP | 2862504 A2 | 22-04-2015 |
| | | | EP | 3081155 A1 | 19-10-2016 |
| | | | HK | 1207811 A1 | 12-02-2016 |
| | | | US | 2008146890 A1 | 19-06-2008 |
| | | | US | 2014051948 A1 | 20-02-2014 |
| | | | US | 2014058220 A1 | 27-02-2014 |
| | | | US | 2014094663 A1 | 03-04-2014 |
| | | | US | 2014235967 A1 | 21-08-2014 |
| | | | US | 2014235968 A1 | 21-08-2014 |
| | | | US | 2014243617 A1 | 28-08-2014 |
| | | | US | 2014323829 A1 | 30-10-2014 |
| | | | US | 2015126825 A1 | 07-05-2015 |
| | | | US | 2015138556 A1 | 21-05-2015 |
| | | | US | 2015150469 A1 | 04-06-2015 |
| | | | US | 2015366471 A1 | 24-12-2015 |
| | | | US | 2018184916 A1 | 05-07-2018 |
| | | | US | 2018220902 A1 | 09-08-2018 |
| | | | US | 2018220903 A1 | 09-08-2018 |
| | | | US | 2018220904 A1 | 09-08-2018 |
| | | | US | 2018220905 A1 | 09-08-2018 |
| | | | US | 2018220906 A1 | 09-08-2018 |
| | | | US | 2018228381 A1 | 16-08-2018 |
| | | | US | 2019357777 A1 | 28-11-2019 |
| | | | US | 2020345243 A1 | 05-11-2020 |
| | | | WO | 2008088511 A1 | 24-07-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **H. W. PAU et al.** Pressure changes in the human middle ear without opening the Eustachian tube. *Acta Oto-Laryngologica,* January 2009, vol. 129 (11), 1182-1186
- **R. J. MARCHBANKS et al.** The effect of raised intracranial pressure on intracochlear fluid pressure: three case studies. *Br J Audiol,* May 1987, vol. 21 (2), 127-130
- **S. E. VOSS et al.** Middle-ear function with tympanic-membrane perforations. I. Measurements and mechanisms. *J. Acoust. Soc. Am.,* September 2001, vol. 110 (3), 1432-1444
- **M. GAIHEDE.** Middle ear volume and pressure effects on tympanometric middle ear pressure determination: model experiments with special reference to secretory otitis media. *Auris Nasus Larynx,* July 2000, vol. 27 (3), 231-239
- **S. E. VOSS ; J. J. ROSOWSKI ; S. N. MERCHANT ; W. T. PEAKE.** Middle-ear function with tympanic-membrane perforations. I. Measurements and mechanisms. *J. Acoust. Soc. Am.,* September 2001, vol. 110 (3), 1432-1444
- **K. TAKEUCHI et al.** Viscoelastic properties of middle ear effusions from pediatric otitis media with effusion and their relation to gross appearance. *Eur Arch Otorhinolaryngol,* January 1990, vol. 247 (1), 60-62